# EUROPEAN PATENT APPLICATION

(11) **EP 3 656 359 A1**
(43) Date of publication of application: **27.05.2020**
(21) Application number: 18207239.7
(22) Date of filing: 20.11.2018
(51) Int. Cl.: A61F 5/02

(54) **LUMBAR SUPPORT BELT**

(71) Applicant: Yang, Chin-Sung, 103 Taipei City (TW)
(72) Inventor: Yang, Chin-Sung, 103 Taipei City (TW)
(74) Representative: Viering, Jentschura & Partner mbB Patent- und Rechtsanwälte

(57) **Abstract**

A lumbar support belt includes a lumbar belt unit (1) and two elastic supporting units (3). The lumbar belt unit (1) has a lumbar belt body (11), two fixing portions (12) and two mounting portions (13). The lumbar belt body (11) has flexibility. The two fixing portions (12) are respectively disposed at two opposite ends of the lumbar belt body (11). The two mounting portions (13) are respectively located between the two opposite ends and the middle portion of the lumbar belt body (11). The two elastic supporting units (3) are respectively disposed on the two mounting portions (13) and can provide an upward supporting force. Each of the elastic supporting units (3) includes a supporting member (31) capable of abutting against an armpit of a user. Accordingly, the weight of the upper body of the user can be transferred to the lumbar belt unit (1) so as to provide support to the lumbar of the user.

## Description

### FIELD OF THE DISCLOSURE

The present disclosure relates to a lumbar support belt, and more particularly to a lumbar support belt that can support and protect the lumbar of a user.

### BACKGROUND OF THE DISCLOSURE

Due to changes of work style and life style in modern society, people often find themselves sitting or standing for long periods of time. Lumbar pains may be caused if good postures are not maintained when sitting or standing for longer periods of time. Conventional lumbar support belts can be used to reduce the stress on the lumbar of a user. The lumbar support belt can be wrapped around the lumbar of the user so as to provide a protecting effect. The middle section of the lumbar support belt is provided with a supporting portion. The two opposite ends of the lumbar support belt is each provided with a retractable portion and a fixing portion. The two retractable portions enable the user to elastically adjust the tightness of the lumbar support belt, and the two fixing portions can be used to fix the lumbar support belt on the lumbar of the user, thereby achieving the effects of tightening the lumbar support belt and supporting the lumbar of the user. However, the conventional lumbar support belt cannot help bear the weight of the upper body of the user so that the lumbar of the user still cannot be well-protected.

In this regard, the present disclosure provides a lumbar support belt to overcome the aforementioned drawbacks.

### SUMMARY OF THE DISCLOSURE

In response to the above-referenced technical inadequacies, the present disclosure provides a lumbar support belt that can help bear the weight of the upper body of a user and provide a good supporting effect for the lumbar of the user.

In one aspect, the present disclosure provides a lumbar support belt that includes a lumbar belt unit and two elastic supporting units. The lumbar belt unit has a lumbar belt body, two fixing portions and two mounting portions. The lumbar belt body is in the form of a belt and has flexibility, the two fixing portions are respectively disposed at two opposite ends of the lumbar belt body, and the two mounting portions are respectively located between the two opposite ends and the middle portion of the lumbar belt body. The two elastic supporting units are respectively disposed on the two mounting portions. Each of the elastic supporting units has elasticity capable of providing an upward supporting force, and each of the elastic supporting units includes a supporting member capable of abutting against an armpit of a user.

The advantages of the present disclosure are that the lumbar belt unit can be wrapped around the lumbar of a user, the supporting members of the two elastic supporting units can respectively abut against the armpits of the user, the elastic supporting units can function as crutches, and the weight of the upper body of the user can be transferred to the lumbar belt unit via the elastic supporting units so as to disperse the weight of the upper body of the user, and provide good support for the lumbar of the user.

In addition, the lumbar belt body of the present disclosure is provided with the airbag, the tightness of the lumbar belt unit can be adjusted by the degree of inflation so as to elastically adjust the tightness of the lumbar belt body according to body shapes of different users. The lumbar belt body of the present disclosure is further provided with the support belt body, and the support belt body can abut against the lumbar of the user, thereby preventing the lumbar support belt from slipping off and providing a good fixing effect.

These and other aspects of the present disclosure will become apparent from the following description of the embodiment taken in conjunction with the following drawings and their captions, although variations and modifications therein may be affected without departing from the spirit and scope of the novel concepts of the disclosure.

### BRIEF DESCRIPTION OF THE DRAWINGS

The present disclosure will become more fully understood from the following detailed description and accompanying drawings.
FIG. 1 is a perspective view showing a lumbar support belt that is in a flattened state according to an embodiment of the present disclosure.
FIG. 2 is an exploded view of FIG. 1.
FIG. 3 is an exploded view showing the lumbar support belt that is in a wrapped state according to the embodiment of the present disclosure.
FIG. 4 is a perspective view showing the lumbar support belt that is in the wrapped state according to the embodiment of the present disclosure.
FIG. 5 is a perspective view showing the lumbar support belt that is in the wrapped state according to the embodiment of the present disclosure from another perspective.
FIG. 6 is a cross-sectional view taken along line VI-VI of FIG. 5.
FIG. 7 is a partial enlarged view of region VII of FIG. 1.
FIG. 8 is a schematic view of another configuration of FIG. 7.
FIG. 9 is a functional block diagram showing a controlling manner of the softness adjusting member according to the embodiment of the present disclosure.
FIG. 10 is a schematic view of the lumbar support belt that is in use according to the embodiment of the present disclosure.
FIG. 11 is a schematic view of the lumbar support belt that is in use according to the embodiment of the present disclosure from another perspective.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an", and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

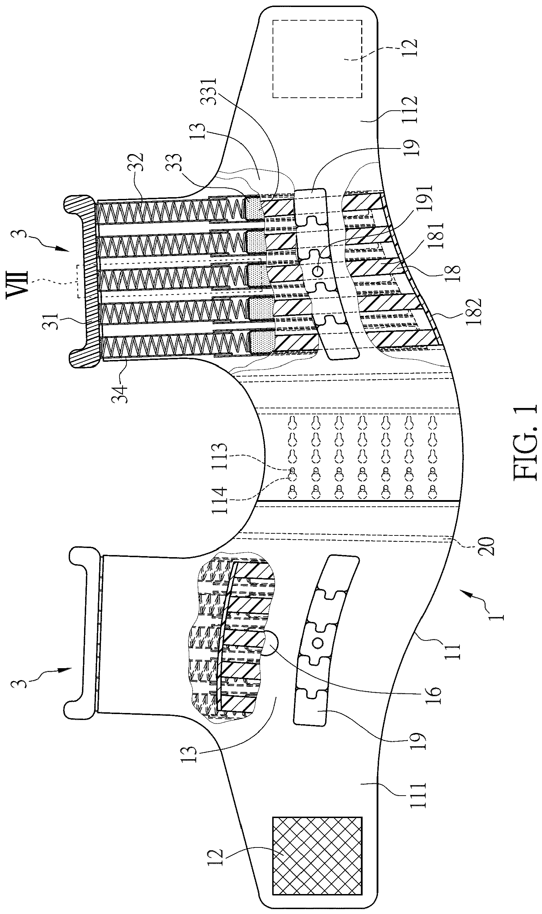

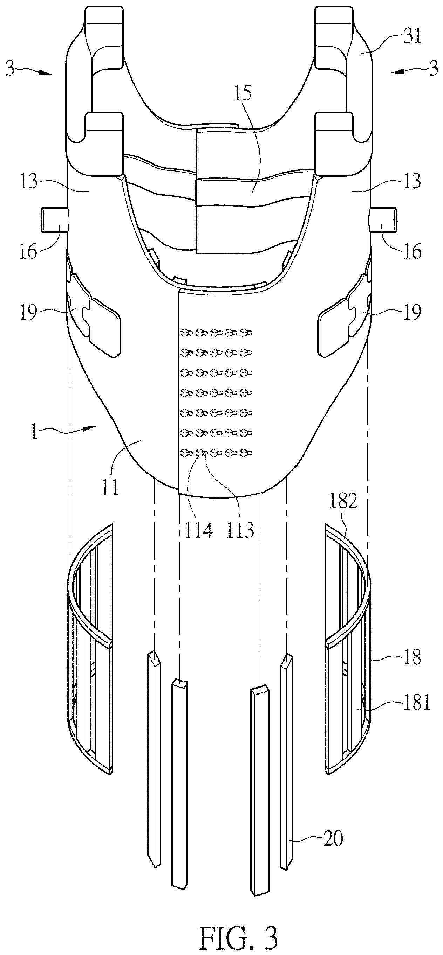

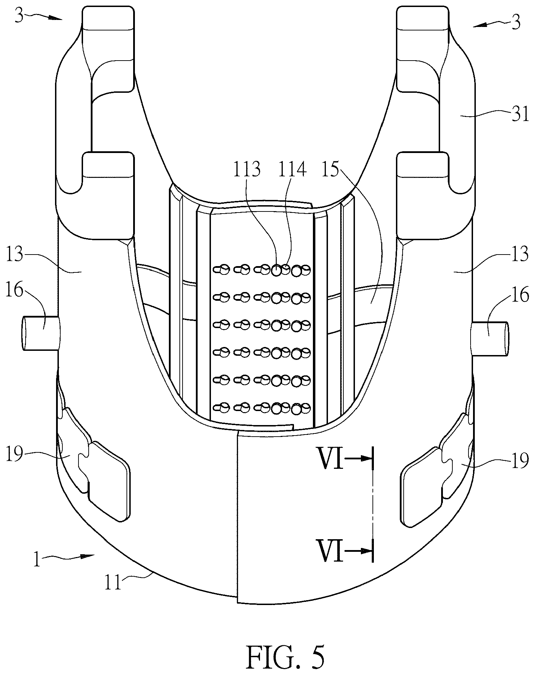

Referring to FIG. 1 to FIG. 6, a preferred embodiment of the present disclosure is illustrated. The various aspects and embodiments disclosed herein are for purposes of illustration and are not intended to be limiting, with the true scope and spirit being indicated by the following claims.

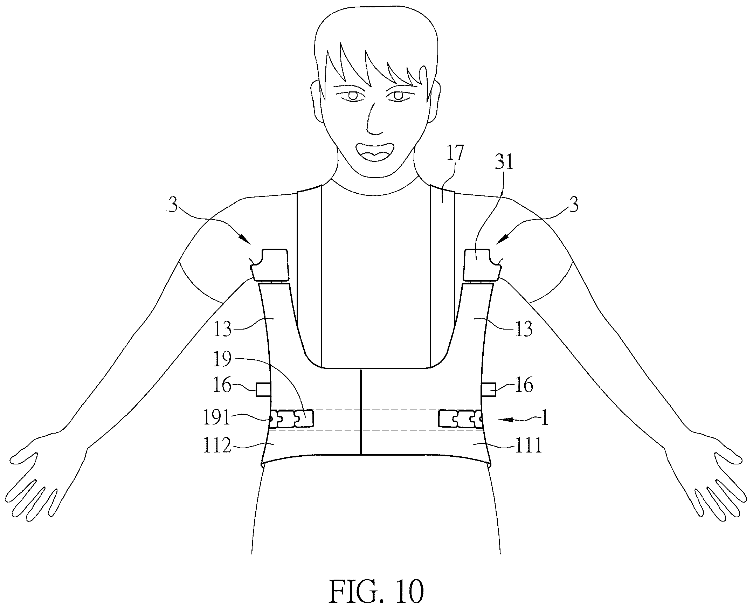

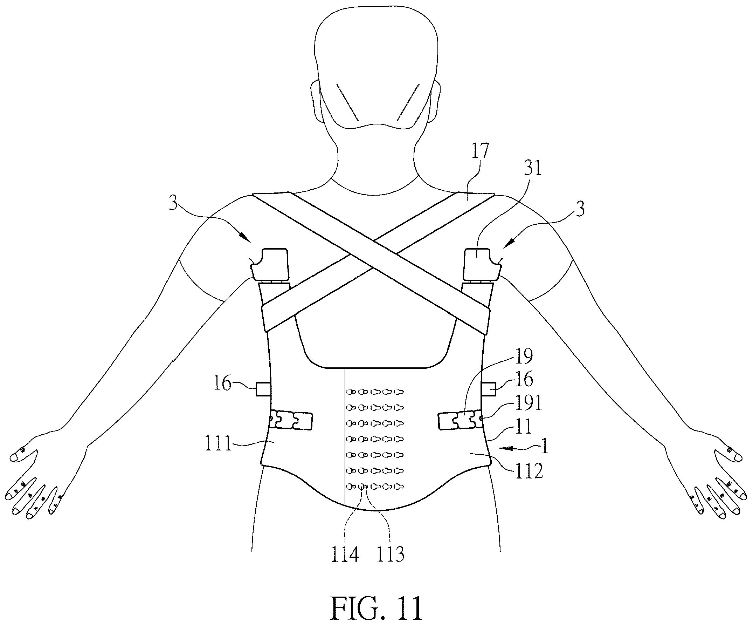

The present embodiment discloses a lumbar support belt which includes a lumbar belt unit 1 and two elastic supporting units 3. The lumbar belt unit 1 has a lumbar belt body 11, two fixing portions 12 and two mounting portions 13. The lumbar belt body 11 is in the form of a belt. An upper edge of the lumbar belt body 11 may be linear or curved, a lower edge of the lumbar belt body 11 may also be linear or curved, and the present disclosure is not limited thereto. The lumbar belt body 11 has flexibility and may have stretchable elasticity such that the lumbar belt body 11 can be bent and wrapped around the lumbar of a user. As shown in FIG. 1, the two fixing portions 12 are respectively disposed at two opposite ends of the lumbar belt body 11 when the lumbar belt body 11 is in a flattened state. In one embodiment of the present disclosure, the two fixing portions 12 may be a male fastening tape and a female fastening tape respectively. In another embodiment of the present disclosure, the two fixing portions 12 may be a male buckle and a female buckle respectively. The two fixing portions 12 can be fixed to each other such that the lumbar support belt can be wrapped around the lumbar of a user as shown in FIG. 10 and FIG. 11.

The two mounting portions 13 are respectively located between the two opposite ends and the middle portion of the lumbar belt body 11. That is, one mounting portion 13 is located between one end and the middle portion of the lumbar belt body 11, and the other mounting portion 13 is located between the other end and the middle portion of the lumbar belt body 11. The two mounting portions 13 each extend upwardly at an appropriate height, such that the two mounting portions 13 both protrude from the upper edge of the lumbar belt body 11, and the two elastic supporting units 3 can be respectively mounted on the two mounting portions 13.

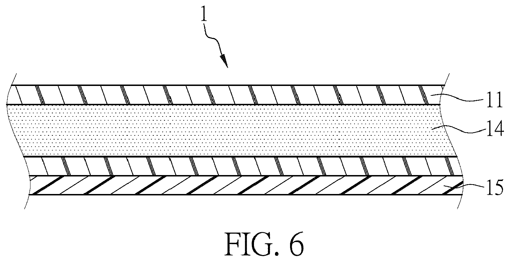

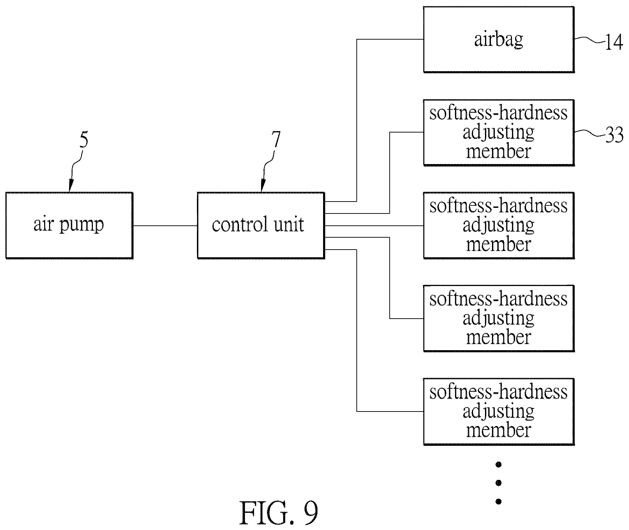

The lumbar belt body 11 may have a two-layer structure or a multi-layer structure. In the present embodiment, the lumbar belt unit 1 further includes at least an airbag 14 as shown in FIG. 6. The airbag 14 is a continuous structure and extends along a length direction of the lumbar belt body 11, but the present disclosure is not limited thereto. For example, in another embodiment of the present disclosure, the airbag 14 includes a plurality of sub-airbags (not shown) spaced apart from each other and arranged along the length direction of the lumbar belt body 11. In addition, the airbag 14 may be disposed in the lumbar belt body 11 (as shown in FIG. 6) or may be disposed outside of the lumbar belt body 11. The airbag 14 may be connected to an air pump 5 (as shown in FIG. 9), and the air pump 5 is capable of inflating or deflating the airbag 14 through a control unit 7. The air pump 5 can be operated electrically or can be operated manually. The air pump is well known to one of skill in the art and need not be reiterated herein. After the airbag 14 is inflated, the tightness of the lumbar belt unit 1 can be adjusted by the degree of inflation so as to elastically adjust the tightness of the lumbar belt body 11 according to body shapes of different users.

The lumbar belt unit 1 further includes a support belt body 15 disposed on the lumbar belt body 11. The support belt body 15 may be made of materials such as leather. The support belt body 15 is exposed outside the lumbar belt body 11. The support belt body 15 may be disposed on a side (i.e. an inner side) of the lumbar belt body 11 close to a human body. The support belt body 15 extends along the length direction of the lumbar belt body 11. When the lumbar support belt is wrapped around the lumbar of a user, the support belt body 15 can abut against the lumbar of the user, thereby preventing the lumbar support belt from slipping off and providing a fixing effect.

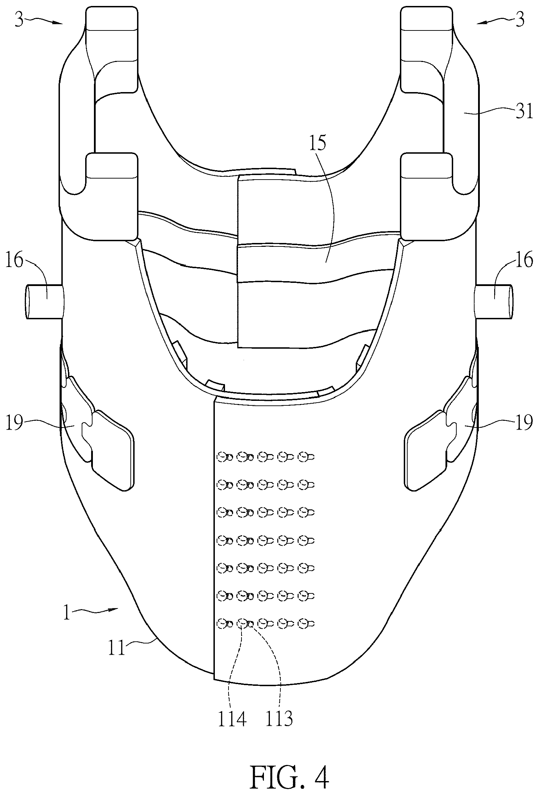

As shown in FIG. 4, the lumbar belt unit 1 further includes two connecting members 16 (i.e. two connecting shafts), and the two connecting members 1 are respectively disposed at two opposite sides of the lumbar belt body 11 when the lumbar belt body 11 is in a wrapped state. The two connecting members 16 can correspond in position to two opposite sides of the lumbar of a user when the lumbar support belt is wrapped around the lumbar of the user. The two connecting members 16 are configured to connect to walking assist devices (not shown) to assist the user in walking. Referring to FIG. 10 and FIG. 11, the lumbar belt unit 1 further includes two shoulder straps 17 respectively disposed on the upper edge of the lumbar belt body 11. The two shoulder straps 17 are configured to be hung from the shoulders of the user to prevent the lumbar support belt from slipping off and to provide a fixing effect.

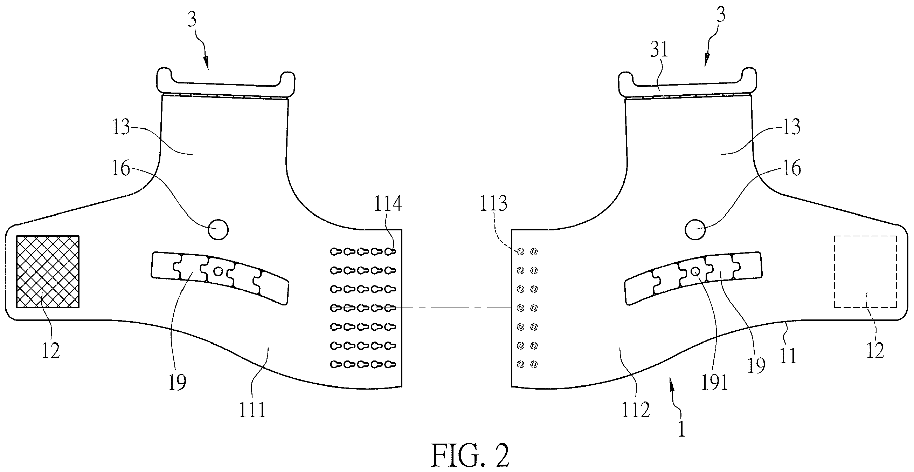

Preferably, the lumbar belt body 11 has a length adjustable mechanism. More specifically, referring again to FIG. 1 and FIG. 2, the lumbar belt body 11 is divided into a left-half section 111 and a right-half section 112. The left-half section 111 and the right-half section 112 are independent belt bodies. The left-half section 111 and the right-half section 112 are respectively provided with a plurality of first fastening portions 113 and a plurality of second fastening portions 114. The first fastening portions 113 may be fastening blocks, and the second fastening portions 114 may be fastening slots. The first fastening portions 113 and the second fastening portions 114 are capable of being selectively engaged with each other such that the length of the lumbar belt body 11 can be elastically adjusted according to body shapes of different users, but the present disclosure is not limited thereto. In another embodiment of the present disclosure, the left-half section 111 may be integrally formed with the right-half section 112.

The lumbar belt unit 1 further includes two steel sheet assemblies 18 and two chain belt bodies 19. The two steel sheet assemblies 18 are respectively located between the two opposite ends and the middle portion of the lumbar belt body 11. The two chain belt bodies 19 are also respectively located between the two opposite ends and the middle portion of the lumbar belt body 11. In the present embodiment, the two steel sheet assemblies 18 are sandwiched between the lumbar belt body 11 having the two-layer structure, the two chain belt bodies 19 are disposed outside of the lumbar belt body 11, and the two steel sheet assemblies 18 respectively correspond in position to the two chain belt bodies 19, but the present disclosure is not limited thereto. Each of the steel sheet assemblies 18 includes a plurality of steel sheets 181 having elasticity. The steel sheets 181 are spaced apart from each other, and the steel sheets 181 are configured to erect the lumbar belt body 11. Each of the steel sheet assemblies 18 further includes two steel strips 182 respectively connected to upper ends and lower ends of the steel sheets 181, such that the steel sheets 181 are integrally connected to each other. The steel sheet assemblies 18 are used to increase the structural strength of the lumbar belt body 11.

Each of the chain belt bodies 19 has a multi-section structure that facilitates bending. The two chain belt bodies 19 are fixed on the lumbar belt body 11, and the two chain belt bodies 19 are respectively located between the two opposite ends and the middle portion of the lumbar belt body 11. The two chain belt bodies 19 respectively extend along the length direction of the lumbar belt body 11. Each of the chain belt bodies 19 is connected to the corresponding steel sheet assembly 18 via a connecting pin 191. The chain belt bodies 19 are used to increase the structural strength of the lumbar belt body 11 and the steel sheet assemblies 18. Moreover, the lumbar belt body 11 may be provided with a plurality of soft steel plates 20 at intervals to further increase the structural strength.

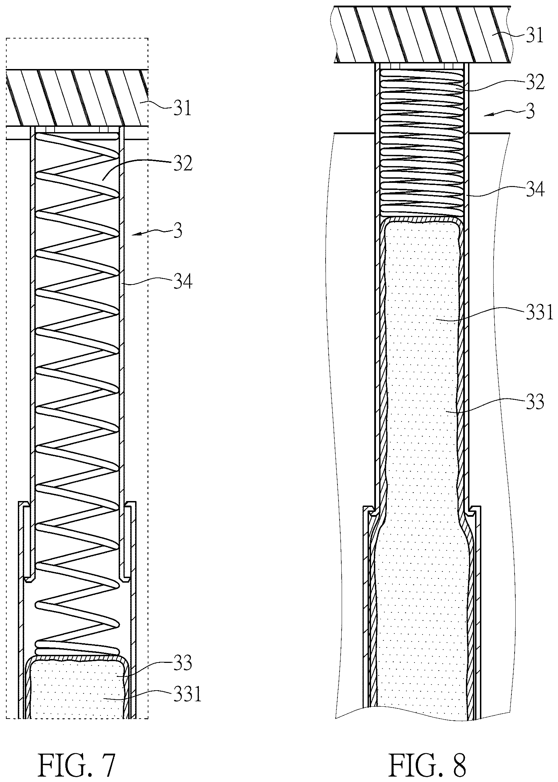

The two elastic supporting units 3 are respectively disposed on the two mounting portions 13. Each of the elastic supporting units 3 has elasticity capable of providing an upward supporting force. Referring to FIG. 7 and FIG. 8, each of the elastic supporting units 3 includes a supporting member 31 capable of abutting against an armpit of a user. Moreover, each of the elastic supporting units 3 further includes a plurality of springs 32 and a plurality of softness adjusting members 33. The springs 32 may be compression springs. In each of the elastic supporting units 3, the springs 32 are disposed on the corresponding mounting portion 13 of the lumbar belt unit 1, and the springs 32 may be respectively accommodated in the corresponding mounting portions 13. The springs 3 are spaced apart from each other. The number of the springs 32 is not limited to those shown in the drawings and may be appropriately increased or decreased according to actual needs. In each of the elastic supporting units 3, the supporting member 31 is connected to upper ends of the springs 32. The supporting member 31 may be a supporting frame with a cushion. A top portion of the supporting member 31 may be curved or have a suitable curvature for appropriately abutting against the armpit of the user.

Each of the softness adjusting members 33 may include various automatic or manual adjusting devices such as an airbag body, a pneumatic cylinder, a hydraulic cylinder or a motor screw. Each of the softness adjusting members 33 may also be adapted to cooperate with a magnet to increase the strength of the upward supporting force. In the present embodiment, each of the softness adjusting members 33 includes an airbag body 331. The airbag bodies 331 respectively abut against the steel sheets 181 such that the steel sheets 181 are capable of supporting the airbag bodies 331. The airbag bodies 331 of the softness adjusting members 33 may be connected to the air pump 5 through pipes (not shown), and the air pump 5 is capable of inflating or deflating the airbag bodies 331 through the control unit 7. In another embodiment of the present disclosure, the air pump 5 can be operated manually to inflate or deflate the airbag bodies 331. In each of the elastic supporting units 3, the softness adjusting members 33 are disposed on the corresponding mounting portion 13 of the lumbar belt unit 1, and the softness adjusting members 33 may be accommodated in the corresponding mounting portion 13. The softness adjusting members 33 are spaced apart from each other. The number of the softness adjusting members 33 is not limited to those shown in the drawings and may be appropriately increased or decreased according to actual needs. In each of the elastic supporting units 3, the softness adjusting members 33 are respectively disposed below the springs 32, and upper ends of the softness adjusting members 33 respectively abut against lower ends of the springs 32.

In the present embodiment, each of the springs 32 and the corresponding softness adjusting member 33 are sleeved by a retractable sleeve 34. That is, each of the springs 32 and the corresponding softness adjusting member 33 are disposed in the corresponding retractable sleeve 34 such that the retractable sleeve 34 is capable of supporting and fixing the spring 32 and the softness adjusting member 33. More specifically, each of the softness adjusting members 33 is capable of being expanded and retracted to change a length and an amount of compression of the corresponding spring 32. When the softness adjusting member 33 is expanded (as shown in FIG. 8), the spring 32 is compressed, the amount of compression of the spring 32 is increased, the length of the spring 32 is decreased, the elasticity of the spring 32 is decreased, and the structure of the spring 32 becomes rigid. Conversely, when the softness adjusting member 33 is retracted (as shown in FIG. 7), the stress of the spring 32 is released, the amount of compression of the spring 32 is decreased, the length of the spring 32 is increased, the elasticity of the spring 32 is increased, and the structure of the spring 32 becomes loose. In addition, the softness adjusting members 33 are electrically connected to the control unit 7 (as shown in FIG. 9), and the softness adjusting members 33 are respectively controlled by the control unit 7. The control unit 7 is a control circuit including an IC board, and the control unit 7 is capable of controlling the softness adjusting members 33 so as to adjust the softness and length of the elastic supporting units 3. The two elastic supporting units 3 are capable of respectively abutting against the armpits of the user (as shown in FIG. 10 and FIG. 11) so that the weight of the upper body of the user can be transferred to the lumbar belt unit 1.

The advantages of the present disclosure are that the lumbar belt unit can be wrapped around the lumbar of a user, the supporting members of the two elastic supporting units can respectively abut against the armpits of the user, the elastic supporting units can function as crutches, and the weight of the upper body of the user can be transferred to the lumbar belt unit via the elastic supporting units so as to disperse the weight of the upper body of the user, and provide good support for the lumbar of the user.

In addition, the lumbar belt body of the present disclosure is provided with the airbag, the tightness of the lumbar belt unit can be adjusted by the degree of inflation so as to elastically adjust the tightness of the lumbar belt body according to body shapes of different users. The lumbar belt body of the present disclosure is further provided with the support belt body, the support belt body can abut against the lumbar of the user, thereby preventing the lumbar support belt from slipping off and providing a good fixing effect.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its spirit and scope.

## Claims

1. A lumbar support belt, comprising:
a lumbar belt unit (1) having a lumbar belt body (11), two fixing portions (12) and two mounting portions (13); wherein the lumbar belt body (11) is in the form of a belt and has flexibility, the two fixing portions (12) are respectively disposed at two opposite ends of the lumbar belt body (11), and the two mounting portions (13) are respectively located between the two opposite ends and the middle portion of the lumbar belt body (11); and
two elastic supporting units (3) respectively disposed on the two mounting portions (13); wherein each of the elastic supporting units (3) has elasticity capable of providing an upward supporting force, and each of the elastic supporting units (3) includes a supporting member (31) capable of abutting against an armpit of a user.

2. The lumbar support belt according to claim 1, wherein each of the elastic supporting units (3) further includes a plurality of springs (32) and a plurality of softness adjusting members (33); wherein in each of the elastic supporting units (3), the springs (32) are disposed on the corresponding mounting portion (13) of the lumbar belt unit (1), the springs (32) are spaced apart from each other, and the supporting member (31) is connected to upper ends of the springs (32); wherein in each of the elastic supporting units (3), the softness adjusting members (33) are disposed on the corresponding mounting portion (13) of the lumbar belt unit (1), the softness adjusting members (33) are spaced apart from each other, the softness adjusting members (33) are respectively disposed below the springs (32), and upper ends of the softness adjusting members (33) respectively abut against lower ends of the springs (32).

3. The lumbar support belt according to claim 2, wherein each of the softness adjusting members (33) includes an airbag body (331), and the softness adjusting members (33) are connected to an air pump (5).

4. The lumbar support belt according to claim 2, wherein each of the springs (32) and the corresponding softness adjusting member (33) are sleeved by a retractable sleeve (34), and each of the springs (32) and the corresponding softness adjusting member (33) are disposed in the corresponding retractable sleeve (34).

5. The lumbar support belt according to claim 1, wherein the lumbar belt unit (1) further includes at least an airbag (14) disposed in the lumbar belt body (11) or disposed outside of the lumbar belt body (11), the airbag (14) extends along a length direction of the lumbar belt body (11), and the airbag (14) is capable of being inflated or deflated such that the tightness of the lumbar belt unit (1) can be adjusted.

6. The lumbar support belt according to claim 1, wherein the lumbar belt unit (1) further includes a support belt body (15) disposed on the lumbar belt body (11), the support belt body (15) is exposed outside the lumbar belt body (11), and the support belt body (15) extends along a length direction of the lumbar belt body (11).

7. The lumbar support belt according to claim 1, wherein the lumbar belt body (11) is divided into a left-half section (111) and a right-half section (112), the left-half section (111) and the right-half section (112) are respectively provided with a plurality of first fastening portions (113) and a plurality of second fastening portions (114), and the first fastening portions (113) and the second fastening portions (114) are capable of being selectively engaged with each other such that the length of the lumbar belt body (11) can be elastically adjusted.

8. The lumbar support belt according to claim 1, wherein the two mounting portions (13) each extend upwardly such that the two mounting portions (13) both protrude from an upper edge of the lumbar belt body (11).

9. The lumbar support belt according to claim 1, wherein the lumbar belt unit (1) further includes two steel sheet assemblies (18) and two chain belt bodies (19), the two steel sheet assemblies (18) are respectively located between the two opposite ends and the middle portion of the lumbar belt body (11), and the two chain belt bodies (19) are also respectively located between the two opposite ends and the middle portion of the lumbar belt body (11); wherein each of the steel sheet assemblies (18) includes a plurality of steel sheets (181), the steel sheets (181) are spaced apart from each other, and the steel sheets (181) are configured to erect the lumbar belt body (11); wherein each of the steel sheet assemblies (18) further includes two steel strips (182) respectively connected to upper ends and lower ends of the steel sheets (181); wherein the two chain belt bodies (19) are fixed on the lumbar belt body (11), and the two chain belt bodies (19) are respectively connected to the two steel sheet assemblies (18).

10. The lumbar support belt according to claim 1, wherein each of the elastic supporting units (3) further includes a plurality of springs (32) and a plurality of softness adjusting members (33); wherein in each of the elastic supporting units (3), the springs (32) are disposed on the corresponding mounting portion (13) of the lumbar belt unit (1), and the supporting member (31) is connected to upper ends of the springs (32); wherein in each of the elastic supporting units (3), the softness adjusting members (33) are disposed on the corresponding mounting portion (13) of the lumbar belt unit (1), each of the softness adjusting members (33) includes an airbag body (331), the softness adjusting members (33) are respectively disposed below the springs (32), and upper ends of the softness adjusting members (33) respectively abut against lower ends of the springs (32); wherein the lumbar belt unit (1) further includes two steel sheet assemblies (18) and two chain belt bodies (19), the two steel sheet assemblies (18) are respectively located between the two opposite ends and the middle portion of the lumbar belt body (11), and the two chain belt bodies (19) are also respectively located between the two opposite ends and the middle portion of the lumbar belt body (11); wherein each of the steel sheet assemblies (18) includes a plurality of steel sheets (181), the steel sheets (181) are spaced apart from each other, and the steel sheets (181) are configured to erect the lumbar belt body (11); wherein each of the steel sheet assemblies (18) further includes two steel strips (182) respectively connected to upper ends and lower ends of the steel sheets (181); wherein the two chain belt bodies (19) are fixed on the lumbar belt body (11), the two chain belt bodies (19) are respectively connected to the two steel sheet assemblies (18), and the airbag bodies (331) respectively abut against the steel sheets (181).
